# EUROPÄISCHE PATENTSCHRIFT

(11) **EP 1 541 158 B1**
(45) Veröffentlichungstag und Bekanntmachung des Hinweises auf die Patenterteilung: **05.03.2008**
(21) Anmeldenummer: 03027836.0
(22) Anmeldetag: 04.12.2003
(51) Int. Cl.: A61K 36/48, A61K 36/185, A61K 36/82, A61K 36/15, A61K 36/87, A61K 36/355

(54) **Antioxidantzubereitung für die orale und/oder topische Anwendung**
Antioxydant composition for oral or topical administration
Composition antioxydante pour l'administration orale ou topique

(43) Veröffentlichungstag der Anmeldung: 15.06.2005
(73) Patentinhaber: Cognis IP Management GmbH, 40589 Düsseldorf (DE); Cognis France, S.A.S., 31360 Boussens (FR)
(72) Erfinder: Buchwald-Werner, Sybille, Dr., 40589 Düsseldorf (DE); Le Hen Ferrenbach, Catherine, 77100 Meaux (FR); Carite, Christophe, 87570 Rilhac-Rancon (FR); Blasquez, José Fernandez, 08228 Terrassa (ES); Rull Prous, Santiago, 08034 Barcelona (ES)

(56) Entgegenhaltungen:
- EP-A- 1 072 265
- EP-A- 1 344 516
- WO-A-02/34072
- US-A1- 2002 155 074

## Beschreibung

### Gebiet der Erfindung

Die Erfindung befindet sich sowohl auf dem Gebiet der Nahrungsmittelzusatz- bzw. Nahrungsmittelergänzungsstoffe als auch auf dem Gebiet der Hautkosmetik und betrifft neue Zubereitungen zur oralen bzw. topischen Anwendung, enthaltend spezielle Wirkstoffmischungen, die das Körpergewebe und insbesondere die Hautzellen stimulieren und Entzündungsherden entgegenwirken.

### Stand der Technik

In den letzten Jahren hat der Markt für Nahrungsmittelzusatzstoffe einen ungeheuren Aufschwung erfahren. Vom Verbraucher werden sowohl Produkte gewünscht, die in einem eher undifferenzierten Ansatz den körperlichen Wohlbefinden nutzen und die Abwehrkräfte steigern, wie dies beispielsweise typisch für Vitamine ist, als auch solche, die unter den Begriffen "Health Food" oder "Dietary Supplements" bekannt sind und beispielsweise den Fettabbau oder den Muskelaufbau beschleunigen. So wird beispielsweise in der internationalen Patentanmeldung WO 97/46230 (WARF) vorgeschlagen, konjugierte Linolsäure für diesen Zweck einzusetzen. Ein weiteres Beispiel für den wachsenden Markt für Nahrungsmittelergänzungsstoffe kann unter der Überschrift "Cosmetic inside" oder "Beauty inside" zusammengefasst werden. Hier geht es darum, Haut und Haare sowie Fingernägel in Ihrer physiologischen Funktion zu unterstützen und Erscheinungen wie z.B. Hautalterung zu verlangsamen. Lange bekannt für solche Anwendungen sind z.B. Carotinoide für den Sonnenschutz.

Aus der EP 1344516 A1 sind Zubereitungen bekannt, welche Tocopherole, OPC-reiche Extrakte der Pflanze *Vitis vinifera* und isoflavonhaltige Extrakte der Pflanze *Trifolium pratense* aufweisen. Gegenstand der EP 1072265 sind Zubereitungen mit einem Gehalt an Tocopherolen, oligomeren Proanthocyanidinen, Isoflavonen und Carotinoiden.

In diesem Zusammenhang besteht jedoch das Bedürfnis, Zubereitungen zur Verfügung zu stellen, die einerseits die einzelnen Aufgabenstellungen im Vergleich zum Stand der Technik in verbesserter Weise lösen - also beispielsweise die gleichen Effekte bei geringerer Dosierung ergeben - als auch unterschiedliche Aufgabenstellungen miteinander verbinden.

Die Aufgabe der vorliegenden Erfindung hat darin bestanden neue Zubereitungen zur Verfügung zu stellen, die sowohl bei oraler Verabreichung im Sinne von Nahrungsmittelergänzungsstoffen (z.B. "Functional Food") als auch bei topischer Anwendung im Sinne eines Hautbehandlungsmittels das Körpergewebe strafft, antioxidativ wirksam ist, den Hormonhaushalt reguliert, die kosmetische Beschaffenheit der Haut verbessert und gleichzeitig über entzündungshemmende Eigenschaften verfügen.

### Beschreibung der Erfindung

Gegenstand der Erfindung sind Zubereitungen für die orale und/oder topische Anwendung, enthaltend
(a) Tocopherole,
(b) Oligomere Procyanolidine (OPC),
(c) Extrakte der Pflanze *Trifolium pratense* bzw. deren aktive Wirkstoffe und
(d) Extrakte der Pflanze *Passiflora incarnata* bzw. deren aktive Wirkstoffe
   sowie gegebenenfalls
(e) Carotinverbindungen.

Überraschenderweise wurde gefunden, dass die quaternären Mischungen, gegebenenfalls um die Komponente (e) ergänzt, ein synergistisches antioxidatives und entzündungshemmendes Verhalten zeigen, den Stoffwechsel im Körper anregen, den weiblichen Hormonhaushalt insbesondere während der Menopause regulieren und insgesamt zu einem strafferen und jugendlicheren Erscheinungsbild der Haut beitragen.

### Tocopherole

Unter dem Begriff Tocopherole, die die Komponente (a) bilden, sind die in 2-Stellung mit einem 4,8,12-Trimethyltridecyl-Rest substituierten Chroman-6-ole (3,4-Dihydro-2H-1-benzopyran-6-ole) zu verstehen, die auch als Biochinone bezeichnet werden. Typische Beispiele sind die Plastichinone, Tocopherolchinone, Ubichinone, Bovichinone, K-Vitamine und Menachinone (z.B. 2-Methyl-1,4-naphthochinone). Vorzugsweise handelt es sich um die Chinone aus der Vitamin-E-Reihe, d.h. α-, β-, γ-, δ- und ε-Tocopherol, wobei letzteres noch über die ursprüngliche ungesättigte prenylseitenkette verfügt (s. Abbildung).

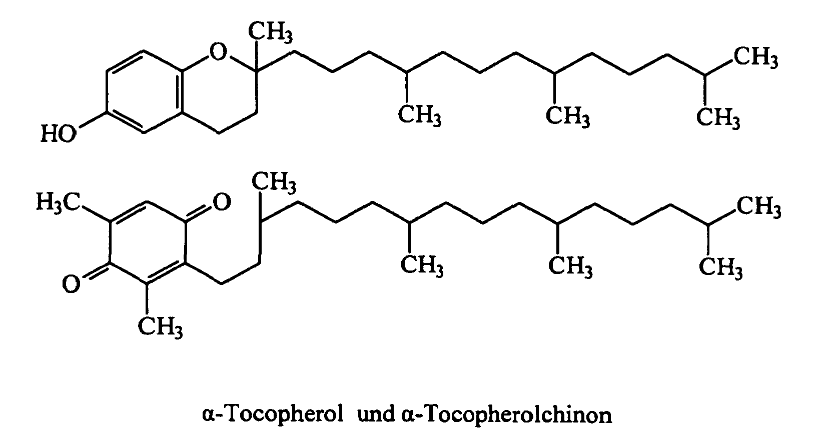

Daneben kommen auch Tocopherolchinone und -hydrochinone sowie die Ester der Chinone mit Carbonsäuren, wie z.B. Essigsäure oder Palmitinsäure in Frage. Der Einsatz von α-Tocopherol, Tocopherolacetat und Tocopherolpalmitat sowie deren Gemische ist bevorzugt.

### Oligomere Procyanolidine (OPC)

Die ersten oligomeren Procyanolidine, die als Komponente (b) in Frage kommen, wurden von Masquellier aus Traubensaaten isoliert. Sie enthalten als Monomerbausteine die im Pflanzenreich weit verbreiteten Tannine. Chemisch betrachtet können zwei Typen von Tanninen unterschieden werden, nämlich kondensierte Formen zu denen auch das Procyanidin A2 gehört, und hydrolysierbare Tannine. Kondensierte Tannine, die auch als Flavolane bezeichnet werden, entstehen in der Biosynthese durch Kondensation von Monomeren, wie z.B. Catechin, Gallocatechin, Afzelechin (2-R, 3-S Typ Monomere) sowie Epicatechin, Epigallocatechin und Epiafzelechin (2-R, 3-R Typ Monomere). Durch Kondensation der Monomeren entstehen zunächst Dimere und dann höhere Oligomere, wobei die Kondensation durch Ausbildung einer C-C-Bindung in 4-8 bzw. 6-8-Position erfolgt. Im Fall der bevorzugten A2-Dimere vom Typ des Proanthocyanidin A2 gibt es eine doppelte Bindung, nämlich C2->O->C7 und C4->C8. Die Struktur ist in der folgenden Abbildung wiedergegeben:

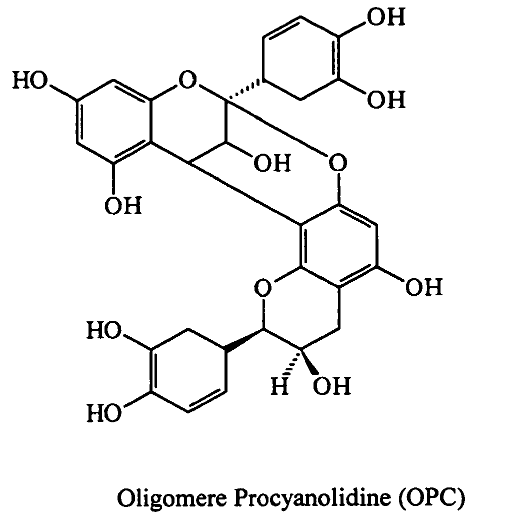

Die A2-Typ Proanthocyanidine sind weniger hydrolyseanfällig als die B-Typen. Im übrigen wird dieser Begriff synonym für die Gruppe der kondensierten Tannine verwendet, da diese unter dem Einfluss heißer Mineralsäuren Monomere abspalten. Die Proanthocyanidine können grundsätzlich synthetischer Natur sein, aus praktischer Sicht kommen jedoch vorzugsweise Anreicherungsprodukte mit einer wirksamen Menge der OPC bzw. A2-Dimeren in Frage, die durch Extraktion von bestimmten Früchten, Saaten, Pflanzen oder Pflanzenteilen gewonnen werden können. Als Quellen kommen insbesondere Grüner Tee *(Camellia sinensis),* Pinienrinde *(Pinia silvestris),* Traubensaat *(Vitis vinifera)* und daneben Litchi pericarp *(Litchi chinensis)* und Potentille *(Potentille erecta)* sowie deren Gemische in Betracht.

### Extrakte der Trifolium pratense

Pflanzenteile und Saaten der Pflanze Trifolium pratense, also des Rotklees, deren Extrakte und aktive Prinzipien die Komponente (c) bilden, sind reich an (Iso-)Flavonen und deren Glucosiden, wie z.B. Daidzein, Genestein, Formononentin und Biochanin A sowie deren Glucoside, wie z.B. Ononin oder Sissostrin :

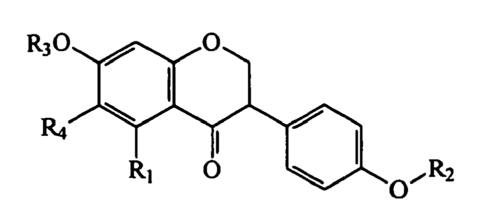

| **Isoflavonglucoside** | **R₁** | **R₂** | **R₃** | **R₄** |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### Extrakte der Passiflora incarnata

Früchte und Saaten der Pflanze *Passiflora incarnata*, also der Passionspflanze, deren Extrakte und aktive Prinzipien die Komponente (d) darstellen, sind reich an Flavonen des Typ Apigenin und Luteolin sowie deren C-Glykosiden :

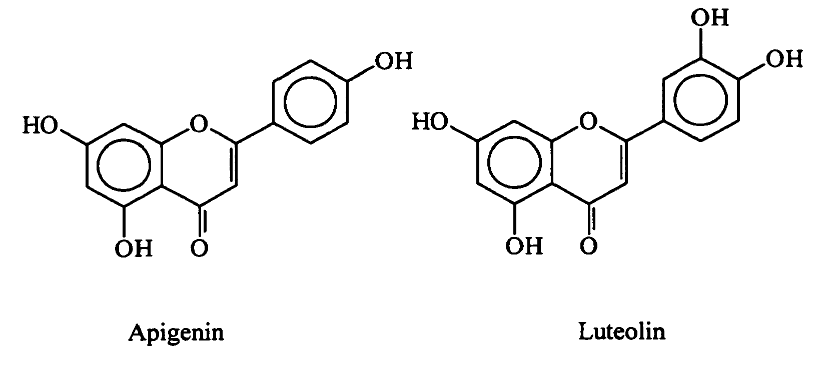

Darüber hinaus enthalten sie 2"-B-D-Glucoside, Schaftoside und Iso-Schaftoside, Vitexin, Isovitexin, Orientin, Isoorientin, Vicenin-2, Incenin-2, Saponanin sowie Spurenelemente, vor allem Calcium, Phosphor und Eisen.

### Extraktion

Die Herstellung der verschiedenen Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegende Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### Carotinverbindungen

Unter Carotinverbindungen, die die optionale Komponente (e) bilden, sind im wesentlichen Carotine und Carotinoide zu verstehen. Carotine stellen eine Gruppe von 11- bis 12fach ungesättigten Triterpenen dar. Von besonderer Bedeutung sind die drei isomeren α-, β- und γ-Carotine, die alle über das gleiche Grundgerüst mit 9 konjugierten Doppelbindungen, 8 Methylverzweigungen (einschließlich möglicher Ringstrukturen) und einer β-Ionon-Ringstruktur an einem Molekülende verfügen und ursprünglich als einheitlicher Naturstoff angesehen worden waren. Nachstehend sind eine Reihe von Carotinverbindungen abgebildet, die als Komponente (b) in Frage kommen, ohne dass es sich um eine abschließende Aufzählung handelt.

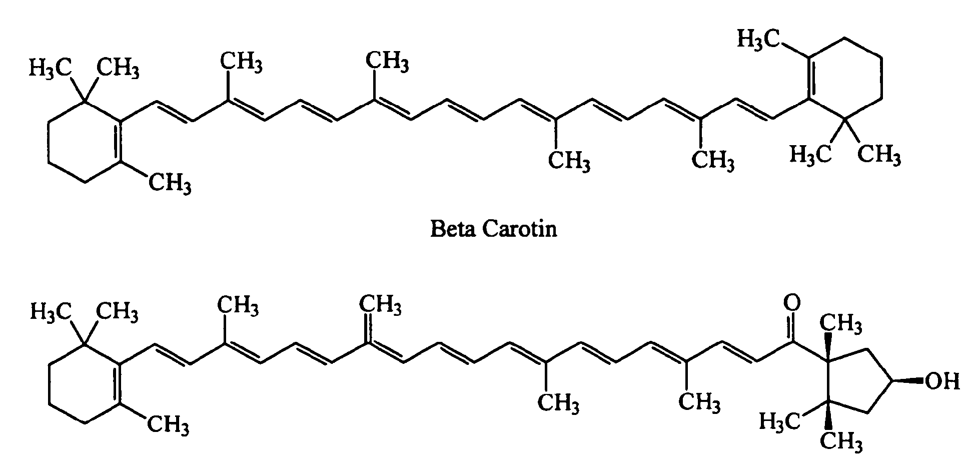

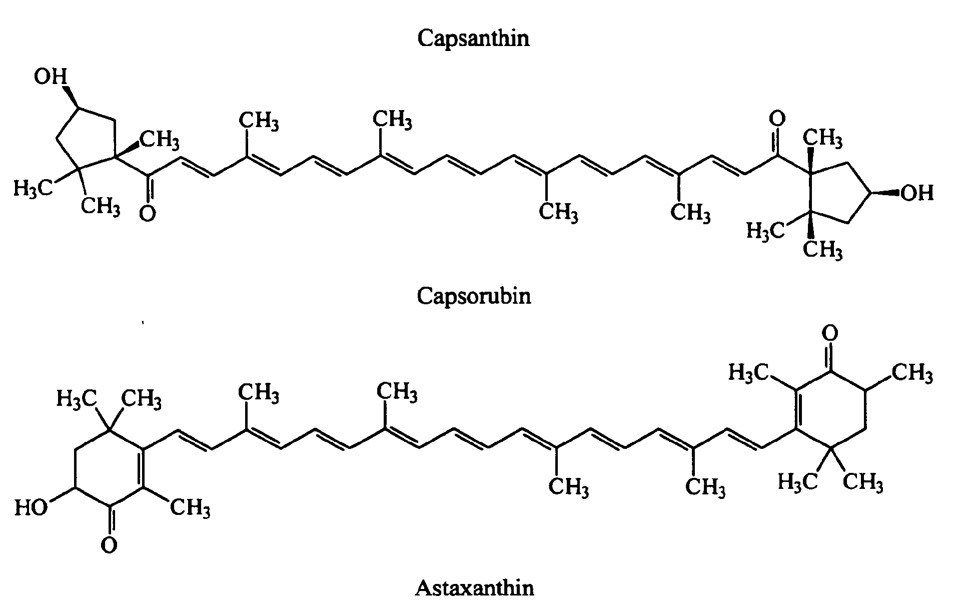

Neben den bereits genannten Isomeren kommen auch das δ-, ε- und ζ-Carotin (Lycopin) in Betracht, wobei freilich das β-Carotin (Provitamin A) wegen seiner hohen Verbreitung von besonderer Bedeutung ist; im Organismus wird es enzymatisch in zwei Moleküle Retinal gespalten. Unter Carotinoiden versteht man sauerstoffhaltige Derivate der Carotine, die auch als Xanthophylle bezeichnet werden, und deren Grundgerüst aus 8 Isopreneinheiten (Tetraterpene) bestehen. Man kann sich die Carotinoide aus zwei C₂₀-Isoprenopiden derart zusammengesetzt denken, dass die beiden mittleren Methylgruppen in 1,6-Stellung zueinander stehen. Typische Beispiele sind das (3R,6'R)-β-ε-Carotin-3,3'-diol (Lutein), (3R,3'S,5'R)-3,3'-Dihydroxy-β,κ-carotin-6-on (Capsanthin), das 9'-cis-6,6'-Diapocarotindisäure-6'-methylester (Bixin), (3S,3'S,5R,5'R)-3,3'-Dihydroxy-κ,κ-carotin-6,6'-dion (Capsorubin) oder das 3S,3'S)-3,3'-Dihydroxy-β,β'-carotin-4,4'-dion (Astaxanthin).

Neben den Carotinen und Carotinoiden sollen unter dem Begriff Carotinverbindungen auch deren Spaltprodukte wie beispielsweise 3,7-Dimethyl-9-(2,6,6-trimethyl-1-cyclohexenyl)-2,4,6,8-nonatetraen-1-ol (Retinol, Vitamin A1) und 3,7-Dimethyl-9-(2,6,6-trimethyl-1-cyclohexenyl)-2,4,6,8-nonatetraenal (Retinal, Vitamin A1-Aldehyd) verstanden werden. In einer bevorzugten Ausführungsform der vorliegenden Erfindung werden als Komponente (a) β-Carotin, Lutein oder deren Gemische im Gewichtsverhältnis 10 : 90 bis 90 : 10 und insbesondere 40 : 60 bis 60 : 40 eingesetzt.

### Zubereitungen für die orale oder topische Anwendung

In einer bevorzugten Ausführungsform der vorliegenden Erfindung können die Zubereitungen die Komponenten (a) bis (e) in folgenden Gewichtsverhältnissen
(a) 10 bis 25, vorzugsweise 5 bis 15 Gew.-% Tocopherole,
(b) 50 bis 80, vorzugsweise 60 bis 70 Gew.-% Oligomere Procyanolidine,
(c) 10 bis 25, vorzugsweise 5 bis 15 Gew.-% Extrakte der Pflanze *Trifolium pratense*,
(d) 1 bis 15 Gew.-% Extrakte der Pflanze *Passiflora incarnata*, sowie
(e) 0 bis 10, vorzugsweise 1 bis 15 Gew.-% Carotinverbindungen
mit der Maßgabe enthalten, dass sich alle Mengenangaben auf den Wirkstoffgehalt beziehen und zu 100 Gew.-% ergänzen.

### Verkapselung

In einer besonderen Ausführungsform der vorliegenden Erfindung werden die oral bzw. topisch anzuwendenden Zubereitungen in verkapselter Form - beispielsweise in Gestalt üblicher Gelatinemakrokapseln - vorzugsweise aber in mikroverkapselter Form eingesetzt.

Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Mikrokapseln und Verfahren zu ihrer Herstellung sind Gegenstand früherer Patenanmeldungen der Patentanmelderin **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a1) aus Gelbildnern, kationischen Polymeren und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Kationpolymerlösungen behandelt und gegebenenfalls dabei die Ölphase entfernt;
oder
(c1) aus Gelbildnem und Wirkstoffen eine Matrix zubereitet,
(c2) die Matrix mit einer Kationpolymerlösung versetzt und
(c3) die Mischung auf einen pH-Wert einstellt der oberhalb des pKₛ-Wertes des Kationpolymers liegt;
oder
(d1) wässrige Wirkstoffzubereitungen mit Ölkörpem in Gegenwart von Emulgatoren zu O/W-Emulsionen verarbeitet,
(d2) die so erhaltenen Emulsionen mit wässrigen Lösungen anionischer Polymere behandelt,
(d3) die so erhaltene Matrix mit wässrigen Kationpolymerlösungen in Kontakt bringt und
(d4) die so erhaltenen Verkapselungsprodukte von der wässrigen Phase abtrennt;
oder
den Wirkstoff abwechselnd mit Schichten aus unterschiedlich geladenen Polyelektrolyten einhüllt (layer-by-layer-Technologie).

### • Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### • Kationische Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguare® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

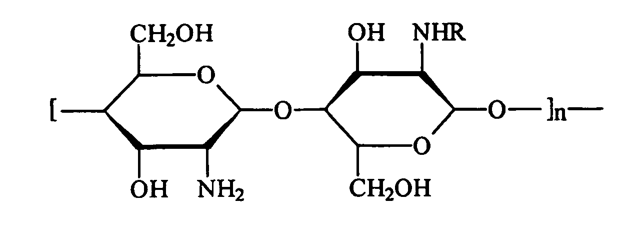

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### • Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglycerid-mischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### • Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

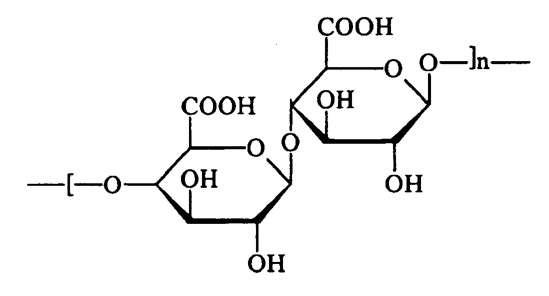

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### • Emulgatoren

Als Emulgatoren kommen anionische, amphotere, kationische oder vorzugsweise nichtionische oberflächenaktive Verbindungen aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole,
- Glycerincarbonat,
- aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure, sowie
- Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent EP 1064088 B1 (Max-Planck Gesellschaft) verwiesen.

### Gewerbliche Anwendbarkeit

Die erfindungsgemäßen Zubereitungen zeichnen sich dadurch aus, dass sie über synergistische antioxidative und entzündungshemmende Eigenschaften verfügen, das Körpergewebe straffen und den Hormonhaushalt regulieren. Weitere Gegenstände der vorliegenden Erfindung betreffen daher die Verwendung der Mischungen als Nahrungsmittelergänzungsstoffe sowie zur Herstellung von Hautbehandlungmitteln, in denen sie in Mengen von 1 bis 10, vorzugsweise 2 bis 8 und insbesondere 3 bis 5 Gew.-% - bezogen auf die Endprodukte - enthalten sein können. Ein letzter Gegenstand der Erfindung betrifft die Verwendung der Zubereitungen zur Herstellung eines Medikamentes zur Regulierung des Hormonhaushaltes im weiblichen Körper während der Menopause.

### Beispiele

### Beispiele 1 und 2. Vergleichsbeispiele V1 bis V8 Wirksamkeit gegenüber freien Radikalen

Die Wirksamkeit der Testsubstanzen gegen freie Radikale wurde nach verschiedenen Methoden sowohl chemisch als auch biochemisch untersucht:
- Methode A: In einem ersten Verfahren wurde Diphenylpicrylhydrazyl (DPPH°) eingesetzt, ein verhältnismäßig stabiles Radikal, welches eine purpurfarbene Lösung ergibt. Bestimmt wurde die optische Dichte (DO) bei 513 nm.
- Methode B: In Gegenwart von Eisen(II)-ionen und EDTA wurden aus Wasserstoffperoxid Hydroxylradikale freigesetzt und zur Oxidation von Desoxyribose verwendet. Das Oxidationsprodukt bildet mit Thiobarbitursäure eine pinkfarbene Verbindung. Dessen Konzentration korrespondiert mit der optischen Dichte bei 532 nm. Untersucht wurde, ob in Gegenwart der Testprodukte weniger Desoxyribose oxidiert, d.h. weniger freie Radikale freigesetzt werden.
- Methode C: Der zuvor geschilderte Versuch wurde in Abwesenheit von EDTA untersucht um die Eignung der Testsubstanzen, inaktive Eisenkomplexe zu bilden, zu überprüfen.
- Methode D: Xanthin Oxidase ist ein Enzym welches infolge oxidativen Stresses ausgeschüttet wird und den Zerfall der Purinbasen Adenin und Guanin in Uronsäure und Superoxidanionen katabolisiert. Letztere dismutieren spontan oder in Gegenwart von Superoxid Dismutase in Wasserstoffperoxid und Sauerstoff. Die Menge an Superoxidanion kann durch NBT Reduktion über die optische Dichte bei 490 nm bestimmt werden. Untersucht wurde, ob in Gegenwart der Testsubstanzen weniger Superoxidanionen generiert bzw. mehr Anionen zerstört werden.

Die Ergebnisse sind in Tabelle 1 zusammengefasst; angegeben sind jeweils die EC50-Werte in % (w/v); d.h. je kleiner die angegebenen Werte sind, umso wirksamer sind die Testsubstanzen. Die Beispiele 1 und 2 sind erfindungsgemäß, die Beispiele V1 bis V8 dienen zum Vergleich.

**Tabelle 1**

| **Antioxidative Wirkung (Angabe in %-rel.)** | | | | | | | | | | |
|---|---|---|---|---|---|---|---|---|---|---|
| **Komponente** | **V1** | **V2** | **V3** | **V4** | **V5** | **V6** | **V7** | **V8** | **1** | **2** |
| Tocopherol | 100 | - | - | 50 | 50 | - | 10 | 15 | 10 | 10 |
| *Vitis* Extrakt | - | - | - | 50 | - | 50 | 80 | 70 | 70 | 70 |
| *Trifolium* Extrakt | - | - | - | - | 50 | 50 | 10 | 15 | 10 | 10 |
| *Passiflora* Extrakt | - | - | - | - | - | - | - | - | 10 | 5 |
| Lutein | - | - | - | - | - | - | - | - | - | 5 |
| Ascorbinsäure | - | 100 | - | - | - | - | - | - | - | - |
| Butylhydroxytoluol | - | - | 100 | - | - | - | - | - | - | - |
| ***Wirkungsnachweis*** | | | | | | | | | | |
| Methode A | 0.007 | 0,001 | 0,050 | 0,008 | 0,009 | - | 0,001 | 0,001 | 0,001 | 0,001 |
| Methode B | n.b. | 0,261 | n.b. | n.b. | n.b. | n.b. | 0,200 | 0,220 | 0,250 | 0,270 |
| Methode C | n.b. | 0,900 | n.b. | n.b. | n.b. | n.b. | 0,750 | 0,750 | 0,790 | 0,850 |
| Methode D | - | 0,591 | - | - | - | - | 0,400 | 0,450 | 0,480 | 0,500 |

Die Ergebnisse lassen den Schluss zu, dass die Testsubstanzen eine antioxidative Wirksamkeit besitzen, die deutlich über der von Tocopherol und BHT und in der gleichen Größenordnung wie Vitamin C liegt. Die Testsubstanzen besitzen in Abwesenheit von EDTA ein besonders hohes Potential zum Quenchen von Hydroxylionen, wodurch nachgewiesen ist, dass sie stabile Eisenkomplexe bilden. Schließlich besitzen sie ein starkes Potential, die Reduktion von BT durch Superoxidanionen zu verhindern.

### Beispiele 3 und 4. Vergleichsbeispiele V9 bis V16 Anti-inflammatorische Wirkung

Im Verlauf einer cutanen Inflammation werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur proinflammatorische Cytokine, Leukotriene und Proteasen, sondern auch ROS, wie z.B. Superoxide und Hypochloritanionen frei, welche die Aufgabe haben, eingedrungene pathogene Keime oder Pilze zu vernichten. Diese Aktivität der PMN während der Inflammation ist als sogenannter Atmungsausbruch ("respiratory burst") bekannt und kann zu zusätzlichen Schäden im Gewebe führen. Zur Untersuchung, inwiefern die Testsubstanzen den Atmungsausbruch verhindern oder mindern können, wurde eine Zelllinie von menschlichen leukemischen Granulocyten dieser PMN zusammen mit jeweils 0,01 % w/v der Testsubstanzen bei 37 °C von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 2 zusammengefasst. Angegeben sind die Zellzahlen sowie die Menge an freigesetzten ROS in %-rel zum Standard als Mittelwert einer Messreihe mit Dreifachbestimmung. Die Beispiele 3 und 4 sind erfindungsgemäß, die Beispiele V9 bis V16 dienen zum Vergleich.

**Tabelle 2**

| **Anti-inflammatorische Wirkung (Angabe in %-reL)** | | | | | | | | | | |
|---|---|---|---|---|---|---|---|---|---|---|
| **Komponente** | **V9** | **V10** | **V11** | **V12** | **V13** | **V14** | **V15** | **V16** | **3** | **4** |
| Tocopherol | - | 100 | 50 | 50 | 50 | - | 10 | 15 | 10 | 10 |
| *Vitis* Extrakt | - | - | 50 | - | - | 50 | 80 | 70 | 70 | 70 |
| *Trifolium* Extrakt | - | - | - | 50 | 25 | 50 | 10 | 15 | 10 | 10 |
| *Passiflora* Extrakt | - | - | - | - | - | - | - | - | 10 | 5 |
| Lutein | - | - | - | - | 25 | - | - | - | - | 5 |
| ***Wirkungsnachweis*** | | | | | | | | | | |
| Zellzahlen | 100 | 98 | 99 | 100 | 99 | 99 | 98 | 97 | 97 | 98 |
| Freigesetzte ROS | 100 | 98 | 98 | 100 | 99 | 100 | 96 | 96 | 56 | 52 |

Die Ergebnisse zeigen, dass die Testsubstanzen insbesondere bei Mitverwendung der Komponente (d) einen starken inhibierenden Einfluss auf den Atmungsausbruch menschlicher Granulocyten besitzen.

### Beispiele 5 und 6, Vergleichsbeispiele V17 bis V24 Wirksamkeit gegen Hautalterung

Das Enzym Glucose-6-phosphat Dehydrogenase (G6PDH) katalysiert den ersten Schritt des sogenannten "Pentose-Wegs", bei dem ein wesentlicher DNA-Baustein, nämlich die Desoxyribose gebildet wird. Dabei wird Glucose-6-phosphat (G6P) mit Hilfe von G6PDH in 6-Phosphatogluconat (6PG) überführt. Gleichzeitig wird das dabei erforderliche Coenzym NADP wird zu NADPH2 reduziert, welches seinerseits eine Vielzahl von anderen biologischen Reaktion wie z.B. das Recycling von Glutathion oder die Lipidsynthese katalysieren kann. Reduziertes Glutathion schützt viele Enzyme, die über SH-Gruppen verfügen und Zellen vor oxidativem Stress, wie beispielsweise UV-Einwirkung. Damit ist der G6PDH-Gehalt ein wichtiger Parameter für den Zellschutz und die die Hautemeuerung. Die G6PDH-Aktivität wurde in-vitro an menschlichen Fibroblasten nach der enzymatischen Methode von *Okada* bestimmt, der DNA-Gehalt nach dem Verfahren von *Desaulniers*. Die Ergebnisse sind in Tabelle 3 zusammengefasst. Angegeben sind die Ergebnisse von jeweils 3 Messreihen mit Dreifachbestimmung in %-rel gegenüber einer Blindprobe; die Einsatzkonzentration hat jeweils 0,01 % w/v betragen. Die Beispiele 5 und 6 sind erfindungsgemäß, die Beispiele V17 bis V24 dienen zum Vergleich.

**Tabelle 3**

| **Stimulierung der G6PDH-Aktivität (Angabe in %-rel.)** | | | | | | | | | | |
|---|---|---|---|---|---|---|---|---|---|---|
| **Komponente** | **V17** | **V18** | **V19** | **V20** | **V21** | **V22** | **V23** | **V24** | **5** | **6** |
| Tocopherol | - | 100 | 50 | 50 | 50 | - | 10 | 15 | 10 | 10 |
| *Vitis* Extrakt | - | - | 50 | - | - | 50 | 80 | 70 | 70 | 70 |
| *Trifolium* Extrakt | - | - | - | 50 | 25 | 50 | 10 | 15 | 10 | 10 |
| *Passiflora* Extrakt | - | - | - | - | - | - | - | - | 10 | 5 |
| Lutein | - | - | - | - | 25 | - | - | - | - | 5 |
| ***Wirkungsnachweis*** | | | | | | | | | | |
| DNA nach 3 d | 100 | 104 | 106 | 106 | 105 | 101 | 118 | 122 | 125 | 133 |
| DNA nach 6 d | 100 | 100 | 102 | 102 | 101 | 100 | 122 | 119 | 124 | 130 |
| G6PDH nach 3 d | 100 | 108 | 106 | 103 | 104 | 101 | 133 | 141 | 149 | 154 |
| G6PDH nach 6 d | 100 | 106 | 106 | 104 | 103 | 103 | 128 | 139 | 142 | 150 |

### Beispiel 13

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 0,5 g Tocopherol (Covitox^{®}, Cognis), 1 g getrockneter *Pinia silvestris* Extrakt, 0,5 g getrockneter *Trifolium pratense* Extrakt (Herbalia^{®} Red Clover, Cognis), 0,2 g getrockneter *Passiflora incarnata* Extrakt (Herbalia^{®} Passiflora, Cognis), 0,5 g Phenonip^{®} und 0,5 g Polysorbat-20 (Tween^{®} 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

### Beispiel 14

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 0,5 g Tocopherol (Covitox^{®}, Cognis), 1 g getrockneter *Pinia silvestris* Extrakt, 0,5 g getrockneter *Trifolium pratense* Extrakt (Herbalia^{®} Red Clover, Cognis), 0,2 g getrockneter *Passiflora incarnata* Extrakt (Herbalia^{®} Passiflora, Cognis), 0,3 g Lutein, 0,5 g Phenonip^{®} und 0,5 g Polysorbat-20 (Tween^{®} 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

In der nachfolgenden Tabelle 4 finden sich eine Reihe von Rezepturen für topisch anzuwendende Hautpflegemittel; dabei steht "C" für Creme und "L" für Lotion.

**Tabelle 4**

| O/W-Pflegeemulsionen | | | | | | | | | | | |
|---|---|---|---|---|---|---|---|---|---|---|---|
| **Komponente** | **1** | **2** | **3** | **4** | **5** | **6** | **7** | **8** | **9** | **10** | **11** |
| | C | C | C | L | C | L | L | C | L | C | C |
| Mikrokapseln gemäß Bsp. 14 | 2.5 | 2 | 3 | 3 | 2 | 2 | 2.5 | 1.7 | 2.5 | 1.5 | 1.2 |
| Eumulgin^{®} VL 75 | | | | | | | | | | 1.5 | |
| Dehymuls^{®} PGPH | | 0.6 | | | | | | | | | |
| Generol^{®} R | | | 0.5 | | | | | | | | |
| Eumulgin^{®} B2 | | | 0.1 | | | | | | | | |
| Tween^{®} 60 | | | | 0.2 | | | | | | | |
| Cutina^{®} E 24 | | | | 0.2 | | | | | | | |
| Hostaphat^{®} KL 340 N | | | | | | | | | 0.5 | | |
| Lanette^{®} E | | | | | | | | 0.6 | | | |
| Amphisol^{®} K | | 0.2 | | | | | | | | | |
| Natriumstearat | | | | | 0.5 | | | | | | |
| Emulgade^{®} PL 68/50 | | | | | | | | | | | 1.2 |
| Tego^{®} Care CG | 0.7 | | | | | | | | | | |
| Tego^{®} Care 450 | | | | | 0.4 | | | | | | |
| Cutina^{®} MD | | 1 | | | 5 | | 2 | | | 6 | |
| Lanette^{®} 14 | | | | 1 | | | | 4 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | 5 | | | | | 4 | 3 | |
| Dow Corning DC^{®} 245 | | | 2 | | 5 | 1 | | | | | |
| Dow Corning DC^{®} 2502 | | | | | 2 | 1 | | | | | 5 |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Veegum^{®} ultra | | | | | | | | | 1 | | |
| Keltrol^{®} T | | | 0.4 | | | | | | 0.5 | | |
| Cosmedia^{®} SP | | 0.3 | | 0.2 | 0.2 | | | | 0.2 | 0.3 | |
| Pemulen^{®} TR 2 | 0.3 | | | | | | | 0.3 | | | |
| Carbopol^{®} Ultrez 10 | | | 0.3 | | | 0.2 | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., pH 6,5 - 7,5 | | | | | | | | | | |

## Patentansprüche

1. Zubereitungen für die orale und/oder topische Anwendung, enthaltend
(a) Tocopherole,
(b) Oligomere Procyanolidine (OPC),
(c) Extrakte der Pflanze *Trifolium pratense* bzw. deren aktive Wirkstoffe und
(d) Extrakte der Pflanze *Passiflora incarnata* bzw. deren aktive Wirkstoffe.

2. Zubereitungen nach Anspruch 1, **dadurch gekennzeichnet, dass** sie als Komponente (a) α-Tocopherol, Tocopherolacetat und Tocopherolpalmitat oder deren Gemische enthalten.

3. Zubereitungen nach den Ansprüchen 1 und/oder 2, **dadurch gekennzeichnet, dass** sie als Komponente (b) oligomere Proanthocyanolidine vom Typ OPC A2 enthalten.

4. Zubereitungen nach Anspruch 3, **dadurch gekennzeichnet, dass** sie als Komponente (b) OPC-reiche Extrakte der Pflanzen *Camellia sinensis, Vitis vinifera* oder *Pinia silvestris* enthalten.

5. Zubereitungen nach mindestens einem der Ansprüche 1 bis 4, **dadurch gekennzeichnet, dass** sie als Komponente (c) Flavone und/oder Isoflavone enthalten.

6. Zubereitungen nach mindestens einem der Ansprüche 1 bis 5, **dadurch gekennzeichnet, dass** sie als aktive Wirkstoffe der *Passiflora incarnata* (Komponente d) Flavone vom Typ Apigenin, Luteolin oder deren C-Glycoside sowie Vitexin und Isovitexin enthalten.

7. Zubereitungen nach mindestens einem der Ansprüche 1 bis 6, **dadurch gekennzeichnet, dass** sie als weitere Komponente (e) Carotinverbindungen enthalten.

8. Zubereitungen nach Anspruch 7, **dadurch gekennzeichnet, dass** als Komponente (e) β-Carotin, Lutein oder deren Gemische enthalten.

9. Zubereitungen nach mindestens einem der Ansprüche 1 bis 8, **dadurch gekennzeichnet, dass** sie
(a) 10 bis 25 Gew.-% Tocopherole,
(b) 50 bis 80 Gew.-% Oligomere Procyanolidine,
(c) 10 bis 25 Gew.-% Extrakte der Pflanze *Trifolium pratense*,
(d) 1 bis 15 Gew.-% Extrakte der Pflanze *Passiflora incarnata*, sowie
(e) 0 bis 10 Gew.-% Carotinverbindungen
mit der Maßgabe enthalten, dass sich alle Mengenangaben auf den Wirkstoffgehalt beziehen und zu 100 Gew.-% ergänzen.

10. Zubereitungen nach mindestens einem der Ansprüche 1 bis 9, **dadurch gekennzeichnet, dass** sie in verkapselter Form vorliegen.

11. Zubereitungen nach Anspruch 10, **dadurch gekennzeichnet, dass** sie als Gelatinemakrokapseln vorliegen.

12. Verwendung von Zubereitungen nach Anspruch 1 als Nahrungsmittelergänzungsstoffe.

13. Verwendung von Zubereitungen nach Anspruch 1 zur Herstellung von Hautschutzmitteln.

14. Verwendung von Zubereitungen nach Anspruch 1 zur Herstellung eines Medikamentes zur Regulierung des Hormonhaushaltes des weiblichen Körpers in der Menopause.

## Claims

1. Preparations for oral and/or topical application containing
(a) tocopherols,
(b) oligomeric procyanolidins (OPCs),
(c) extracts of the plant *Trifolium pratense* or active components thereof and
(d) extracts of the plant *Passiflora incamata* or active components thereof.

2. Preparations as claimed in claim 1, **characterized in that** they contain α-tocopherol, tocopherol acetate and tocopherol palmitate or mixtures thereof as component (a).

3. Preparations as claimed in claims 1 and/or 2, **characterized in that** they contain oligomeric proanthocyanolidins of the OPC A2 type as component (b).

4. Preparations as claimed in claim 3, **characterized in that** they contain OPC-rich extracts of the plants *Camellia sinensis, Vitis vinifera* or *Pinia silvestris* as component (b).

5. Preparations as claimed in at least one of claims 1 to 4, **characterized in that** they contain flavones and/or isoflavones as component (c).

6. Preparations as claimed in at least one of claims 1 to 5, **characterized in that** they contain the flavones apigenin, luteolin or mixtures thereof and vitexin and isovitexin as active components of plant *Passiflora incamata* (component d).

7. Preparations as claimed in at least one of claims 1 to 6, **characterized in that** they contain carotene compounds as an additional component (e).

8. Preparations as claimed in claim 7, **characterized in that** they contain β-carotene, lutein or mixtures thereof as component (e).

9. Preparations as claimed in at least one of claims 1 to 8, **characterized in that** they contain
(a) 10 to 25% by weight tocopherols,
(b) 50 to 80% by weight oligomeric procyanolidins,
(c) 10 to 25% by weight extracts of the plant *Trifolium pratense,*
(d) 1 to 15% by weight extracts of the plant *Passiflora incarnata* and
(e) 0 to 10% by weight carotene compounds,
with the proviso that the all the above quantities are based on the active component content and add up to 100% by weight.

10. Preparations as claimed in at least one of claims 1 to 9, **characterized in that** they are present in encapsulated form.

11. Preparations as claimed in claim 10, **characterized in that** they are present as gelatine macrocapsules.

12. Use of the preparations claimed in claim 1 as food supplements.

13. Use of the preparations claimed in claim 1 for the production of skin protection preparations.

14. Use of the preparations claimed in claim 1 for the production of a medicament for regulating the hormone balance of the female body in the menopause.

## Revendications

1. Préparations pour l'administration orale et/ou topique renfermant :
(a) des tocophérols,
(b) des procyanolidines oligomères (OPC),
(c) des extraits de la plante *Trifolium pratense* ou leurs substances actives,
(d) des extraits de la plante *Passiflora incarnata* ou leurs substances actives.

2. Préparations selon la revendication 1,
**caractérisées en ce qu'**
elles renferment en tant que composant (a) de l'α-tocophérol, de l'acétate de tocophérol et du palmitate de tocophérol ou leurs mélanges.

3. Préparations selon les revendications 1 et/ou 2,
**caractérisées en ce qu'**
elles renferment en tant que composant (b) des proanthocyanolidines oligomères de type OPC A2.

4. Préparations selon la revendication 3,
**caractérisées en ce qu'**
elles renferment, en tant que composant (b) des extraits riches en OPC des plantes *Camellia sinensis*, *Vitis vinifera ou Pinia silvestris.*

5. Préparations selon au moins l'une des revendications 1 à 4,
**caractérisées en ce qu'**
elles renferment en tant que composant (c) des flavones et/ou isoflavones.

6. Préparations selon au moins l'une des revendications 1 à 5,
**caractérisées en ce que**
en tant que substances actives de la *Passiflora incarnata* (composant d) elles renferment des flavones de type apigénine, lutéoline ou leurs C-glycosides ainsi que de la vitexine et de l'isovitexine.

7. Préparations selon au moins l'une des revendications 1 à 6,
**caractérisées en ce qu'**
elles renferment, en tant qu'autre composant (e) des composés du carotène.

8. Préparations selon la revendication 7,
**caractérisées en ce qu'**
elles renferment en tant que composant (e) du β-carotène, de la lutéine ou leurs mélanges.

9. Préparations selon au moins l'une des revendications 1 à 8,
**caractérisées en ce qu'**
elles renferment :
(a) 10 à 25 % en poids de tocophérols,
(b) 50 à 80 % en poids de procyanolidines oligomères,
(c) 10 à 25 % en poids d'extraits de la plante *Trifolium pratense,*
(d) 1 à 15 % en poids d'extraits de la plante *Passiflora incarnata*, et
(e) 0 à 10 % en poids de composés du carotène.
étant précisé que toutes les données quantitatives se rapportent à la teneur en substances actives et se complètent à 100 % en poids.

10. Préparations selon au moins l'une des revendications 1 à 9,
**caractérisées en ce qu'**
elles se présentent sous forme encapsulée.

11. Préparations selon la revendication 10,
**caractérisées en ce qu'**
elles se présentent sous la forme de macrocapsules de gélatine.

12. Utilisation de préparations selon la revendication 1 en tant que compléments alimentaires.

13. Utilisation de préparations selon la revendication 1 pour la préparation d'agents de protection de la peau.

14. Utilisation de préparations selon la revendication 1 pour l'obtention d'un médicament pour réguler la teneur hormonale du corps féminin à la ménopause.
